# EUROPEAN PATENT SPECIFICATION

(11) **EP 1 140 081 B1**
(45) Date of publication and mention of the grant of the patent: **11.08.2010**
(21) Application number: 99966359.4
(22) Date of filing: 17.12.1999
(51) Int. Cl.: A61K 31/425, A61K 31/44, A61K 31/42

(54) **IMPROVING MENTAL PERFORMANCE BY INCREASING BRAIN INSULIN SENSITIVITY**
VERBESSERUNG DER GEISTIGEN FÄHIGKEITEN DURCH DIE STEIGERUNG DER INSULIN-EMPFINDLICHKEIT DES GEHIRNS
UTILISATION ACCRUE DE GLUCOSE PAR LE CERVEAU

(30) Priority: 17.12.1998 US 112669 P; 24.02.1999 US 122258 P
(43) Date of publication of application: 10.10.2001
(60) Divisional application: 08014071.8
(73) Proprietor: Mindset Biopharmaceuticals (USA), Inc., New York, NY 10018 (US)
(72) Inventor: CHAIN, Daniel G., 93227 Jerusalem (IL); CAWTHORNE, Mike, Buckingham MK 18 1EG (GB)
(74) Representative: Grünecker, Kinkeldey, Stockmair & Schwanhäusser Anwaltssozietät
(86) International application number: PCT/US1999/030066
(87) International publication number: WO 2000/035437

(56) References cited:
- WO-A-00/23407
- WO-A-00/23415
- WO-A-00/23416
- WO-A-00/23417
- WO-A-00/23445
- WO-A-00/23451
- WO-A-00/32190
- WO-A-97/31907
- WO-A-99/16758
- WO-A-99/20614
- WO-A-99/38850
- US-A- 4 540 564
- US-A- 5 039 794
- US-A- 5 556 843
- US-A- 6 028 088
- COMBS, C.K. ET AL: "Inflammatory mechanisms in Alzheimer's disease: Inhibition of beta.amyloid -stimulated proinflammatory responses and neurotoxicity by PPAR.gamma agonists" THE JOURNAL OF NEUROSCIENCE, vol. 20, no. 2, 15 January 2000 (2000-01-15), pages 558-567, XP000933946
- KITAMURA, Y. ET AL: "Increased expression of cyclooxygenases and peroxisome proliferator-activated receptor.gamma in Alzheimer's disease brains" BIOCHEMICAL AND BIOPHYSICAL RESERACH COMMUNICATIONS, vol. 254, no. 3, 27 January 1999 (1999-01-27), pages 582-586, XP000929650 cited in the application
- DUELLI, R. ET AL: "Intracerebroventricular injection of streptozotocin induces discrete local changes in cerebral glucose utilization in rats" INTERNATIONAL JOURNAL OF DEVELOPMENTAL NEUROSCIENCE, vol. 12, no. 8, 1994, pages 737-747, XP000933903 cited in the application
- MUKHERJEE, R. ET AL: "Sensitization of diabetic and obese mice to insulin by retinoid X receptor agonists" NATURE, vol. 386, 1997, pages 407-410, XP002081440 cited in the application
- BLUM-DEGEN D. ET AL: "Altered regulation of brain glucose metabolism as a cause of neurodegenerative disorders?" JOURNAL OF NEURAL TRANSMISSION, vol. suppl. 46, 1995, pages 139-147, XP000933904 cited in the application
- GRANNEMAN, J. ET AL: "Member of the Peroxisome Proliferator-Activated Receptor family of transcription factors is differentially expressed by oligodendrocytes" JOURNAL OF NEUROSCIENCE RESEARCH, vol. 51, 1998, pages 563-573, XP000933914 cited in the application
- HOYER, S. ET AL: "Brain glucose metabolism is controlled by amplification and desensitization of the neuronal insulin receptor" ANNALS OF THE NEW YORK ACADEMY OF SCIENCES, vol. 777, 1996, pages 374-379, XP000933907 cited in the application

## Description

### CROSS-REFERENCE TO RELATED APPLICATIONS

### FIELD OF THE INVENTION

The present invention is directed to the use of an agent that improves insulin sensitivity of peripheral tissues in non-insulin dependent diabetic patients and is useful in treating non-insulin diabetes
for the preparation of a pharmaceutical composition
for treating Alzheimer's disease, memory loss, senile dementia, or dementia, when the symptoms of reduced mental performance are not related to non-insulin dependent diabetes or a state of general impaired glucose tolerance,
with the proviso that the use is not the use of a thiazolidinedione for the preparation of a pharmaceutical composition for treating Alzheimer's disease.

### BACKGROUND OF THE INVENTION

Glucose is a major energy source for cerebral tissue. Glucose transported to the cerebral tissue is metabolized by hexokinase to glucose-6-phosphate, which is a very important intermediate in the glucose catabolism system. After this, the glucose-6-phosphate enters a metabolic pathway where it is catabolized in order to generate high-energy phosphate compounds, such as ATP, through its linked phosphorylation reaction.

There is a strong linkage between brain glucose utilization and mental performance as illustrated by the use of PET scans as an aid to identifying brain injury and in evaluating new therapies for senile dementia. It has generally been believed that brain glucose utilization is not affected by changes in circulating insulin concentration and changes in the normal diet or eating pattern. It has been argued that a function critical to maintaining life cannot be dependent upon such things as the nature and timing of meals. Indeed, measurements of whole brain glucose utilization do not show any significant overall differences in relation to diet, insulin treatment or diabetes. The ability to measure brain glucose utilization was originally developed by Sokolov and colleagues, *cf*. Dienel et al (1992). The principle was developed for animal studies and used radiolabelled 2-deoxyglucose, which is taken up by cells through the glucose transport system, is converted to 2-deoxyglucose-6- phosphate, but cannot be further metabolized. Further, the glucose-6-phosphate only minimally accumulates in the brain. Thus, measurement of trapped 2-deoxyglucose-6-phosphate can be used as an index of brain glucose utilization. In animals, this involves direct extraction of tissues. In humans, 18-fluoro-2- deoxyglucose is used, and this can be detected by PET scanning.

These techniques have been used in both animals and humans to demonstrate that the rate of glucose utilization is not uniform throughout the brain. Furthermore, certain portions and cell types, such as astrocytes, are clearly insulin sensitive (Clarke et al, 1984). Astrocytes are important communicating cells, and the insulin sensitivity of these cells can be demonstrated by in vitro studies (Clarke et al, 1984). Diabetes severely affects glucose utilization by astrocytes, and it has also been shown that astrocytes can develop insulin resistance.

*In vivo* studies in rats have shown that glucose utilization is reduced 50% by anesthetic, and that overall glucose utilization by insulin-resistant fa/fa rats is less than in lean littermates. Corticosterone decreased glucose utilization in the limbic regions, but increased utilization in thalamic regions. Adrenalectomy increased glucose utilization in limbic regions. Minipump infusion of insulin for four days to create hyperinsulinaemia reduced glucose utilization in the suprachiasmic nucleus (affecting circadian rhythm), locus coeruus and basolateral amygdala. All of these studies indicate that glucose utilization in discrete areas of the brain is under complex control and can be modified by circulating hormones and possibly by nutrients (Doyle et al, 1995).

The development of memory loss and dementia is generally an age-related process. It is well known that insulin-stimulated fuel utilization in peripheral tissues, such as skeletal muscle, often declines with age and insulin resistance develops. The nature of the insulin resistance is generally related to a reduced amplification of the insulin signaling process, so that insulin is less efficient in activating the normal insulin response. Glucose utilization in the brain also decreases with age (Smith and Sokoloft in The Aging Brain). This might suggest that insulin resistance in discrete brain areas and cell types may play a role in central glucose utilization, resulting in reduced mental performance. On the other hand, as glucose utilization in the brain is believed to be under complex control, one cannot predict whether or not insulin resistance plays a major role in age- decreased brain glucose utilization.

A number of therapies have been developed to inhibit memory loss and dementia in aging patients. However, none of these therapies depends upon controlling insulin action in the brain.

Duelli et al (1994) disclose that a decrease in cerebral glucose utilization may correspond to changes in morphobiological parameters which have been found in patients with Alzheimer's Disease. However, there is no indication that any type of treatment of this decrease in cerebral glucose utilization could be used to prevent or treat Alzheimer's Disease.

Blum-Degen et al (1995) disclose that there is a measurable decrease of *in vivo* and post-mortem cerebral glucose metabolism, and that insulin plays an important role in regulating brain glucose homeostasis in the central nervous system. It has been suggested that the reduction of brain glucose metabolism in neuro-degenerative disorders may be related to a defect of the neuronal insulin-insulin receptor interaction. However, there is no specific disclosure of treating neuro-degenerative disorders with substances which improve insulin sensitivity.

Hoyer et al (1996) disclose that intracerebroventricularly-administered insulin exerts anabolic effects on cerebral glucose/energy metabolism. However, there is no indication that administering a substance to improved insulin sensitivity could be useful in treating age-related brain disorders, such as dementia.

Dore et al, (1997) disclose that insulin receptor sites are not markedly altered during the normal aging process in the Long Evans rat, despite significant learning deficits in memory- impaired aged animals. This suggests that insulin action in the brain does not contribute to the age-related memory loss.

Cullingford (1998) reported that PPAR (peroxisome proliferator-activated receptor) gamma mRNA was expressed at a similar level to PPAR alpha mRNA in adult rat astrocytes, but there is no indication that this receptor might be involved with regulating insulin action and glucose metabolism in this cell type.

Granneman et al (1998) show that PPAR is strongly expressed in immature oligodendrocytes, suggesting a role in oligodendrocyte differentiation. One thazolidinedione compound, Pfizer CP68722, increased the number of oligodendrocytes in glial cultures. However, there is no indication of a connection between this work and improved central glucose utilization.

Romeo et al, in U.S. Patent 5,556,843, disclose that phosphoryl-L-serine-N-acyl-sphingosine can increase glycemia and histamine levels, resulting in cerebral glucose accumulation. This compound can be used in therapies associated with a slowdown of cerebral metabolism, in involutive cerebral syndromes of different origins, and Alzheimer's Disease, memory loss, senile dementia, etc. Phosphoryl-L-serine-N-acyl-sphingosine is said to be useful in treating insulin-dependent diabetes. However, there is no indication that any other drug used for treating diabetes could be used to treat senile dementia or other types of neurological conditions. In fact, phosphoryl-L-serine-N-acyl-sphingosine has no connection to insulin action or insulin resistance.

Ohmoto et al, in U.S. Patent 5,710,153, disclose tetrazole compounds which are used in treating, among other conditions, insulin-dependent diabetes and neural diseases, such as Alzheimer's Disease and Parkinson's Disease. This treatment depends on inhibition of ICE enzymatic activation which leads to prevention of conversion of pre-IL-1β to IL-1β and presumably relates to preventing the inflammatory action of IL-1β. Thus, these compounds have no correlation to insulin action or insulin resistance.

Guthikonda et al, in U.S. Patent 5,629,322 disclose cyclic amidine analogs as inhibitors of nitric oxide synthase which can be used to treat Type I diabetes and dementia. However, there is no indication that there is any effect on glucose metabolism directly from these compounds. In fact, the inhibitors of nitric oxide synthase are associated with inflammatory reactions in insulin-dependent diabetes rather than glucose utilization.

Shapiro, in U.S. Patent 5,668,117, discloses treating neurological diseases, such as diabetic neuropathy, with at least one carbonyl trapping agent, alone or in combination with a therapeutically effective co-agent. However, there is no indication that the carbonyl trapping agent acts by increasing glucose metabolism. The carbonyl trapping agents can be used with insulin derivatives and other conventional medicaments for treating diabetes.

Torii et al, in U.S. Patent 5,693,614, disclose the use of α-FGF for treating senile dementia of the ischemic and hypoglycemic types, stating that the failure of energy metabolism in the brain resulting from hypoglycemia elicits this type of lesion formation. However, α-FGF is not related to any insulin- stimulated process, and there is no indication that this is a conventional treatment for diabetes which can then be used to treat hypoglycemic dementia.

Nakagama et al (1996) disclose that TAK-147, a novel acetylcholinesterase inhibitor when given daily for 40 days to aged rats, increased energy metabolism and increased brain glucose utilization. However, there is no indication that this compound has any effect whatsoever on insulin sensitivity, nor does it act at the PPAR gamma receptor.

Jannetta, in U.S. Patent No. 5,962,004, discloses neurogenic diabetes mellitus can be treated by relieving pressure from a region of the brainstem within the cranium of a patient with a neuroendocrine servomechanism. By relieving pressure on the neuroendocrine servomechanism, the disease is ameliorated. However, this has nothing to do with increasing brain function.

Kumagai, in Diabetes Metab. Res. Rev. 15(4):261-273, Jul-Aug, 1999, discloses that neural tissue is entirely dependent on glucose for normal metabolic activity, and that metabolism in the brain is dependent upon adequate glucose delivery from the systemic circulation. Changes in endothelial glucose transport may have profound consequences on glucose delivery to these tissues and major implications in the development of two major diabetic complications, namely, insulin-induced hypoglycemia and diabetic retinopathy.

Hasselbalch et al., in Diabetes 48:(10): 1915-1921, October, 1999, disclose that hyperinsulinaemia within the normal physiologic range does not affect blood brain barrier glucose transport or net cerebral glucose metabolism. However, there is nothing in this article that deals with increasing brain glucose metabolism.

Another problem associated with treating dementia by increasing brain glucose metabolism is that for patients with normal serum glucose it is important to treat only brain glucose metabolism. Accordingly, it is important to deliver agents for treating brain glucose metabolism to the cerebral tissue. Unfortunately, the blood brain barrier is the major obstacle for delivering most medication to the central nervous system. The capillaries in the brain parenchyma possess high-resistance, tight junctions between the endothelial cells. The cells also lack pores, so that the brain capillary endothelium behaves like a continuous lipid bilayer. Diffusion through this bilayer, the physical blood brain barrier, is largely dependent on the lipid solubility of the solute. Water-soluble molecules (such as glucose, essential amino acids, glutamate) enter the brain almost exclusively by carrier-mediated transport.

Various strategies have been applied to direct medications specific for the central nervous system into the brain. An invasive procedure that includes surgical implantation of an intraventricular catheter followed by pharmaceutical infusion into the ventricular compartment delivers a metabolically unstable compound only to the surface of the brain, *cf*. Poplack et al (1981). Transient opening of the tight junctions of the intracarotid infusion of an osmotically active substance (e.g., mannitol, arabinose) in high concentrations (>1M) may facilitate an indiscriminate delivery of molecules that otherwise cannot cross the blood brain barrier, as shown in Neuwelt et al (1984). However, this procedure is accompanied by severe toxic effects which can lead to inflammation, encephalitis, and seizures. These invasive procedures are only justified for some life-threatening conditions and are not acceptable for less serious illness.

A non-invasive method for peptide delivery into the central nervous system has been suggested that uses the formation of chimeric peptides, *cf*. Pardridge (1986). This strategy relies on the presence of specific receptor-mediated transcytosis systems in the blood brain barrier for certain larger peptides, such as insulin, insulin-like growth factor, transferrin, and albumin. Covalently coupling (e.g., via disulfide bonds) a non- transportable peptide to these transport vectors results in a chimeric peptide that can also undergo receptor-mediated transcytosis, and the active peptide can be released by its enzymatic cleavage in the central nervous system. However, these carriers are not brain-specific, as uptake by non-neural cells or cells outside the central nervous system has been shown. Low amounts of the peptide relative to the carrier molecule, as well as the receptor-based cellular transport mechanism that has physiologically limited transporter capacity (saturable) also prevent pharmacologically significant amounts from entering the brain. Finally, release of the active peptide from the conjugate has not been documented.

Another method for peptide delivery is a simple pharmacologically based approach in which peptide "prodrugs" are administered that are lipophilic esters or amides of the molecule (Tsuzuki et al, 1991). Although the acquired lipophilicity of these prodrugs may assure penetration of the blood brain barrier, as well as other membranes, this is not the sole factor involved in the transport of a peptide into the central nervous system. Blood brain barrier transport of cyclosporin, which is one of the most lipid soluble peptides, is paradoxically low because of peptide degradation, *cf*. Beagley et al, 1990).

A dihydropyridine-pyridinium redox system has recently been successfully applied to delivery to the brain of a number of drugs. Generally speaking, according to this system, a dihydropyridine derivative of a biologically active compound is synthesized, which derivative can enter the central nervous system through the blood-brain barrier following its systemic administration. Subsequent oxidation of the dihydropyridine species to the corresponding pyridinium salt leads to delivery of the drug to the brain.

Four main approaches have been used thus far for delivering drugs to the brain using a redox system. The first approach involves derivation of selected drugs which contain a pyridinium nucleus as an integral structural component. This, approach was first applied to delivering to the brain methylpyridinium-2-carbaldoxime chloride (2-PAM), the active nucleus of which constitutes a quaternary pyridinium salt, by way of the dihydropyridine latent-initiated prodrug form thereof. Thus, a hydrophilic compound (2-PAM) was made lipoidal by making its dihydropyridine form (Pro-2-PAM) to enable its penetration through lipoidal barriers. This simple prodrug approach allowed the compound to get into the brain, as well as into other organs. However, this manipulation did not and could not result in any brain specificity. On the contrary, such approach was limited to relatively small molecule quaternary pyridinium ring-containing drug species, and did not provide the overall ideal result of brain-specific, sustained release of the desired drug, with concomitant rapid elimination from the general circulation, enhanced drug efficacy and decreased toxicity. There was no "trapping" in the brain of the 2-PAM formed *in situ*, and consequently there was no sustained delivery of the 2-PAM. The 2- PAM was eliminated as quickly from the brain as from the general circulation and other organs, *cf*. U.S. Patents 3,929,813 and 3,962,447, and 5,624,894.

Subsequent extension of this approach to delivering a much larger quaternary salt, berberine, to the brain via its dihydropyridine prodrug form was, however, found to provide site- specific, sustained delivery to the brain of that anticancer agent, *cf*. Bodor et al (1981). However, this approach is limited to delivery of active quaternary pyridinium salts.

A second approach for delivering drugs to the brain using a redox system involves the use of a dihydropyridine/ pyridinium carrier chemically linked to a biologically active compound. Bodor et al (1981) outlines a scheme for this specific and sustained delivery of drug species to the brain, as depicted in the following Scheme A:

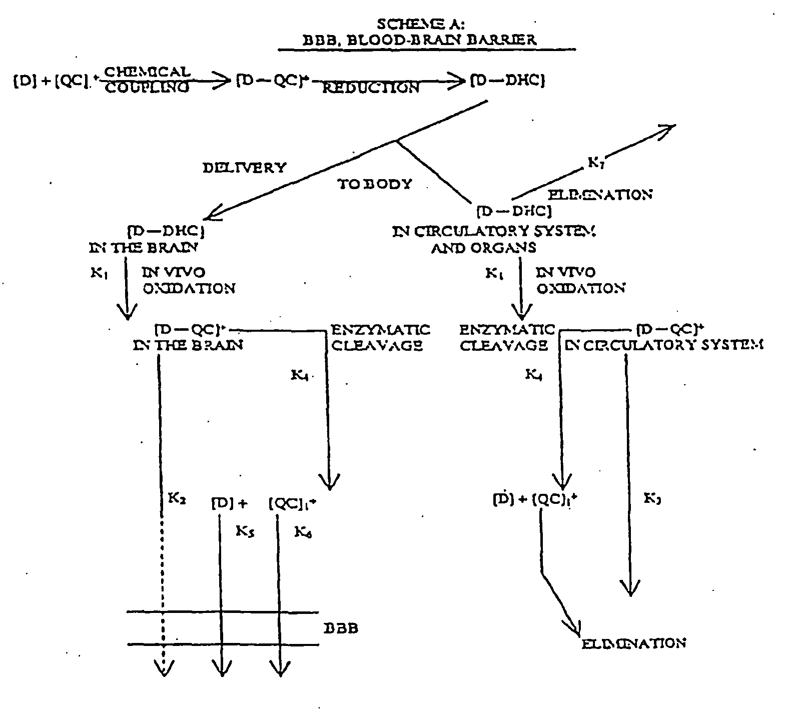

According to the scheme, a drug [D] is coupled to a quaternary carrier [QC]⁺ and the resulting [D-QC]⁺ is then chemically reduced to the lipoidal dihydro form [D-DHC]. After administration of [D-DHC] *in vivo*, it is rapidly distributed throughout the body, including the brain. The dihydro form [D- DHC] is then *in situ* oxidized (rate constant k₁) by the NAD-NADH system to the ideally inactive original [D-QC]⁺ quaternary salt which, because of its ionic hydrophilic character, should be rapidly eliminated from the general circulation of the body, while the blood-brain barrier should prevent its elimination from the brain (K₃>>K₂; K₃>>K₇). Enzymatic cleavage of the [D-QC]⁺ that is "locked" in the brain effects a sustained delivery of the drug species [D], followed by its normal elimination (K₅), metabolism. A property selected carrier [QC]⁺ will also be rapidly eliminated from the brain (K₆>>K₂). Because of the facile elimination of [D-QC]⁺ from the general circulation, only minor amounts of drug are released in the body (K₃>>K₄) ; [D] will be released primarily in the brain (K₄>K₂). The overall result ideally is a brain-specific sustained release of the target drug species.

Specifically, Bodor et al worked with phenylethylamine as the drug model. That compound was coupled to nicotinic acid, then quaternized to give compounds that were subsequently reduced by sodium dithionite. Testing of the N-methyl derivative *in vivo* supported the criteria set forth in Scheme A. Bodor et al speculated that various types of drugs might possibly be delivered using such carrier systems and indicated that use of N- methylnicotinic acid esters and amides and their pyridine ring- substituted derivatives was being studied for delivery of amino- or hyroxyl-containing drugs, including small peptides, to the brain. No other possible specific carriers were disclosed. Other reports of this work with the redox carrier system have appeared, particularly as disclosed in U.S. Patent 4,430,601 and Bodor et al (1983) and Bodor U.S. Patent 4,540,564.

The U.S. Patent 4,540,564 specifically discloses applying the dihydropyridine-pyridinium salt carrier system to amino acids and peptides, particularly small peptides having 2 to 20. amino acid units. Thus, in the carrier system applied to amino acids and peptides, the free carboxy function is protected in an effort to prevent premature metabolism, e.g., with an ethyl ester, while the trigoneline-type carrier is linked to the amino acid or peptide through its free amino function. Oxidation of the dihydropyridine carrier moiety *in vivo* to the ionic pyridinium salt carrier/drug entity prevents elimination thereof from the brain, while elimination from the general circulation is accelerated. Subsequent cleavage of the quaternary carrier/drug species results in sustained delivery of the amino acid or peptide (e.g., tryptophan, GABA, leu-enkaphalin, etc.) to the brain and facile elimination of the carrier moiety. This method is quite useful for delivery of amino acids. In the case of peptides, however, the typical suggested carboxy protecting groups do not confer sufficient lipophilicity to the peptide molecule. Moreover, this approach neither addresses the problem of the enyzmatic blood-brain barrier nor suggests a means of avoiding that problem.

The third approach for delivering drugs to the brain using a redox system provides derivatives of centrally acting amines in which a primary, secondary or tertiary amino function has been replaced with a dihydropyridirie/pyridinium salt redox system. These brain-specific analogs of centrally acting amines have been described in U.S. Patents 4,771,059, 5,082,853 and 5,296,483. The dihydropyridine analogs act as a delivery system for the corresponding biologically active quaternary compounds *in vivo*. Due to its lipophilic nature, the dihydropyridine analog will distribute throughout the body and has easy access to the brain through the blood brain barrier. Oxidation *in vivo* then provides the quaternary form, which is preferentially "locked" in the brain. In U.S. contradistinction to the drug-carrier entities described in Patent 4,540,564 and related publications, however, there is no readily metabolically cleavable bond between drug and quaternary portions, and the active species delivered is not the original drug from which the dihydro analog was derived, but rather is the quaternary analog itself.

In the analog systems described in Bodor U.S. Patents 5,082,853, 4,771,059;and 5,296,483, as applied to amino acids and peptides, the free carboxyl function is, thus, protected to prevent premature metabolism while the dihydropyridine-pyridinium salt type redox system replaces the free amino function in the amino acid or peptide.

As described in the above Bodor patents, the chemical processes for preparing the redox analog derivative replace any free amino function in the selected drug with the redox analog system. When these processes are applied to amino acids, they provide a redox amino acid which no longer contains a free amino function for linkage to another amino acid or peptide via a peptide bond (-CONH-). Such an analog amino acid can, thus, only be used to prepare a peptide having the analog amino acid located at the peptide's N-terminus. This limits use of the redox analog amino acids in peptide synthesis. Moreover, this approach is not designed to deliver the original peptide to the brain, since there is no cleavable bond between peptide and quaternary portions. Rather, the redox portion in this approach becomes an inherent; essentially inseparable, part of a new peptide analog. Furthermore, this approach does not address the problem of the enzymatic blood-brain barrier or suggest a means for avoiding the premature degradation caused by the highly active neuropeptide degrading enzymes.

The fourth redox approach is designed to provide redox amino acids which can be used to synthesize peptides having a redox analog system inserted at a variety of locations in the peptide chain, including non-terminal positions, and has been described in Bodor U.S. Patent 4,888,427. These amino acids contain a redox system appended directly or via an alkylene bridge to the carbon adjacent to the carboxyl carbon.

However, this fourth redox approach, like the third approach discussed above, is not designed ultimately to deliver the original peptide to the brain because there is no cleavable bond between the peptide and the quaternary portions. Again, the redox system becomes an integral part of a new peptide analog, not a means for ultimately delivering the original peptide to the brain. Moreover, this approach also does not address the problem of the enzymatic blood brain barrier or suggest a means for avoiding deactivation of the peptide by enzymes before it achieves its therapeutic objectives.

WO 0032190 forms prior art under Article 54 (3) EPC and discloses methods and compositions for treating Alzheimer's disease and conditions with an inflammatory component. Subject matter of WO 0032190 is excluded by way of disclaime from the present claims.

### SUMMARY OF THE INVENTION

It is an object of the present invention to overcome the aforementioned deficiencies in the prior art.

It is another object of the present invention to increase brain glucose utilization in circumstances where brain glucose utilization is sub-optimal for normal mental performance by increasing insulin sensitivity of insulin-sensitive cells in the brain.

It is a further object of the present invention to prevent and treat age-related memory loss and dementia.

It is another object of the present invention to act on the nuclear receptors PPAR gamma, PPAR delta, and PPAR alpha to prevent or reduce age-related memory loss and dementia.

It is still another object of the present invention to provide a means for delivering drugs which increase brain glucose utilization directly to the central nervous system.

It is another object of the present invention to provide drugs which increase brain glucose utilization in the central nervous system in the form of prodrugs.

According to the present invention, brain glucose utilization can be increased by administering an agent that improves central nervous system insulin sensitivity. By improving insulin sensitivity in the central nervous system, brain glucose utilization is improved and age-related memory loss and dementia can be prevented and/or reduced. More specifically, compounds that interact with the PPAR gamma PPAR delta and PPAR alpha receptors, such as thiazolidinedione and other agents that improve insulin sensitivity, are used to treat senile dementia, Alzheimer's Disease and other dementias, as well as to improve mental performance. These compounds are known to improve insulin sensitivity of peripheral tissues in non-insulin-dependent diabetic patients and are useful in treating non-insulin-dependent diabetes. It has now, surprisingly, been found that the same compounds improve glucose utilization in the central nervous system, leading to improved mental performance, particularly in patients who are otherwise not sensitive to insulin.

More specifically, the present invention provides, in one embodiment, administering certain known thiazolidinedione compounds and derivatives thereof and/or non-thiazolidinedione insulin sensitizers/anti- hyperglycemic agents and derivatives thereof, to an individual who is neither in a state of non-insulin-dependent diabetes (NIDD) nor in a state of general impaired glucose tolerance, but who has symptoms of reduced mental performance. Any pharmaceutical useful in improving insulin sensitivity in non-insulin dependent diabetes patients would be expected to be useful in improving the mental capacity of patients who are neither in a state of NIDD or in a state of general impaired glucose performance.

A number of compounds for the treatment of NIDD have been developed or are under development. These compounds include thiazolidinediones, including troglitazone (Sankyo, Warner- Lambert/Parke Davis, Glaxo), rosiglitazone (Smith Kline Beecham) and pioglitazone (Takeda, Lilly). Other useful compounds include oxyzolidinediones, such as JTT 501 (Japan Tobacco), and non-chiral acyclic agents, such as GW 262570 (Glaxo Wellcome). All of these agents act on the nuclear receptor PPAR gamma. According to the present invention, these compounds may also be used to improve central nervous system insulin sensitivity in non-NIDD patients who have symptoms of reduced mental performance. Willson et al., in WO 97/31907 describe substituted 4-hydroxy-phenylalcanoic acid derivatives with agonist activity to PPAR gamma which can be used to improve central nervous system insulin sensitivity in NON-IDD patients in need thereof.

It is believed that activation of PPAR gamma receptors is the basis of the metabolic action. PPAR gamma receptors form heterodimers with RxR receptors and the heterodimers interact with PPAR response elements to regulate transcription. Thus, activators for the RxR receptors are also insulin sensitizers (Mukherjie et al, 1997).

Petrova et al., in Proc. Natl. Acad. Sci. USA 96(8):4668-73, April 13, 1999, and Kitamura et al., Nuerosci. Lett. 262(2):129-32, March 5, 1999, note that the peroxisome proliferator-activated receptor-gamma (PPAR-gamma)in glial cells is activated by anti-diabetic thiazolidinediones as well as by natural ligands. Kitamura et al. in Biochem. Biophys. Res. Comm 1999 January 27, 254(3):582-6, found that in brains of patients with Alzheimer's disease, PPAR gamma level was increased in the cytosotic fraction but not in the particulate fraction. These results suggest that PPAR gamma activators have inhibitory effects on inflammatory events in the brains of patients with Alzheimer's disease.

### DETAILED DESCRIPTION OF THE INVENTION

Brain glucose utilization is improved, either in the entire brain, or in discrete areas or in specific cell types, by administering an agent that improves insulin sensitivity in the brain. Specific types of cells affected include astrocytes, which are major communicating cells, glial cells and cells in the blood brain barrier. Specific areas of the brain affected which affect memory loss and dementia are the blood brain barrier microvessels and/or the areas of the brain associated with mental performance or memory.

Of particular importance in improving brain glucose utilization are thiazolidinediones, such as troglitazone, rosiglitazone, pioglitazone, darglitazone and englitazone and derivatives thereof. Other types of agents that have been found to improve brain glucose utilization are oxyzolidinediones, such as JTT 501, and non-chiral acyclic agents, such as GW 262570, as well as substituted 4-hydroxy-phenylalcanoic acid derivatives with agonist activity to PPAR gamma. More specific types of compounds which improve brain glucose utilization are compounds which activate the PPAR gamma receptor, or where the agent has agonist or partial agonist activity at the PPAR gamma receptor.

Among the thiazolidinedione compounds which have been found to improve brain glucose utilization are those described in Olefsky et al, U.S. Patents 5,478,852 and 5,708,012. Additional such thiazolidinedione derivatives are disclosed in Antonucci et al, U.S. Patents 5,457,109 and 5,602,133, the entire contents of which are hereby incorporated by reference, including all references cited therein.

Substituted thiazolidinedione derivatives, such as those disclosed in Hindley et al U.S. Patents 5,464,169 and 5,756,525, and in Cantello et al (1994), are also expected to be useful in improving brain glucose utilization and, thus, mental performance in patients in need thereof.

Non-thiazolidinedione insulin sensitizer agents, such as those disclosed by Buckle et al (1996a and 1996b) and substituted 4-hydroxy-phenylalcanoic acid derivatives, such as those described in Willson et al. patent number WO 9731907, are also expected to improve brain glucose utilization, and, thus, mental performance in patients in need thereof.

Hypoglycemic alkaloids, such as quindoline and cryptolepine, which may be obtained from extracts from *Cryptolepsis sp*., as disclosed in Luo et al U.S. Patent 5,629,319, as well as triterpenoid substances, such as those disclosed in Inman et al U.S. Patent 5,691,386, and eremophilanolide sesquiterpenes, such as described in Inman et al U.S. Patent 5,747,527, can also be used to treat or prevent dementia and memory loss in accordance with the present invention.

Other compounds for improving insulin sensitivity are disclosed in Vyas et al U.S. Patent 5,700,820; Ubillas et al U.S. Patent 5,674,900; and Dominianni et al U.S. Patent 5,641,796. These compounds include polymorphic forms of troglitazone, terpenoid-type quinones and C-substituted pentacycloazoles and N-alkyl substituted pentacycloazoles.

Other types of agents which improve brain glucose utilization for improving insulin sensitivity are those which selectively activate one of the sub-types of the human PPAR gamma receptor, namely PPAR gamma 1 and PPAR gamma 2, or where the agent activates a RxR receptor that forms a heterodimer with a PPAR gamma receptor, for example, ligand 100268, which is an RxR receptor ligand.

In addition to the compounds enumerated above, the agent can be a natural product, such as extract of *Crytolepsis* or derived from a natural product such as cryptoleptine.

More specifically, compounds which activate the PPAR gamma receptor or which have agonist activity at the PPAR gamma receptor are useful in improving insulin sensitivity, thereby improving central glucose utilization and ameliorating the memory loss and dementia associated with Alzheimer's Disease and various other types of dementia. Additional types of compounds which are useful include those which activate the PPAR delta receptor or the PPAR alpha receptor.

For purposes of the present invention, "derivative" of the compounds enumerated means chemical modifications of the active compounds which substantially retain the desired activity of the compounds, i.e., the activity of improving central nervous system insulin sensitivity and increasing brain glucose utilization.

Strategies have been specifically tailored to achieving delivery of an agent directly to the central nervous system, i.e., to crossing the blood brain barrier, based upon "sequential metabolism" of a prodrug (*cf*. Bodor et al, 1992; Prokai et al, 1994; Bodor et al, 1995). In one embodiment of a prodrug form of an agent according to the present invention, the modifying group comprises an alkyl ester to facilitate blood-brain barrier permeability.

In one embodiment of the present invention, the compounds for activation of PPAR gamma receptors or which have agonist activity at the PPAR gamma receptor are delivered across the blood brain barrier by packaging the compounds in a molecular environment which disguises the nature of the compound. This environment provides a biolabile, lipophilic function to penetrate the blood brain barrier by passive transport.

In another embodiment of the present invention, the pharmacologically active molecule provides a dihydropyridine-type redox moiety for targeting the compound to the brain and providing "lock-in" as the pyridinium salt. A spacer is placed between the redox moiety and compound designed to enhance the sequential metabolism of the "molecularly packaged" compound. The spacer may be an amino acid or di- or tri-peptide spacer, or any other short group that is compatible with the compound to be administered and that can readily be cleaved *in vivo* to release the active compound.

In another embodiment of the present invention, a cell scattering factor, hereinafter denoted "egressin", isolated from a clone derived from a human metatstaic melanoma (M3827), as described in Wier et al U.S. Patent 5,039,794, is used to aid in transporting drugs across the blood brain barrier to increase glucose metabolism in the central nervous system. Egressin disperses endothelial cells and acts on the cell-cell junctions of the endothelial cells lining the blood vessels. The dissociation of these junctions increases the permeability of the vessels and decreases the effectiveness of the blood brain barrier. Thus, egressin aids in transporting drugs for improving glucose utilization across the blood brain barrier for treating the central nervous system.

In this embodiment, an amount of egressin effective for the dissociation of the cell-cell junction of endothelial cells is administered intravenously along with a therapeutically effective compound for improving glucose utilization in the brain.

Another approach for enabling the compounds used in the present invention to cross the blood brain barrier is to formulate the compounds such that the compounds are non-ionic when they reach the blood stream and would be subsequently enzymatically oxidized to release the compound in a therapeutic amount at the brain. These prodrugs include compounds which contain a tertiary nitrogen atom exhibiting a low basicity, i.e., a pKa below 7.4. On this basis, the skilled artisan can readily appreciate that such compounds, when introduced into the bloodstream of a warm- blooded animal whose pH is about 7.4, would remain essentially non-ionic, thus permitting the prodrug form to be highly protein bounded and/or enter the erythrocytes and, thus, to pass through the blood brain barrier and be subsequently enzymatically oxidized to release the active compound in high bioavailability at the brain.

Where appropriate, the compounds can be provided in the acid addition salt form for the sole purpose of rendering stability to the prodrug free base form prior to administration. Once the prodrug compounds are administered by any route, the salt moiety is "cleaved", thus releasing the remaining tertiary amine form, which readily transcends the blood brain barrier. Next, the prodrug can be enzymatically oxidized to the active compound in an analogous manner to the nicotinamide dinucleotide co-enzyme mediated oxido-reduction system, wherein the coenzyme, in turn, is reduced to the dihydro form (NADH). For purposes of the present invention, the term "prodrug" refers to a derivatized form of a proven drug which, when administered to an individual, is enzymatically or otherwise acted upon in the bloodstream to release the drug at the therapeutic site or sites of activity. In the present invention, the therapeutic site is the central nervous system.

The compounds used to increase glucose utilization in the brain can be provided in the form of prodrugs to alter the biodistribution of the compounds, e.g., to allow compounds which would not typically cross the blood-brain barrier to cross the blood-brain barrier. For example, an anionic group, e.g., a sulfate or sulfonate, can be esterified, e.g., with a methyl group or a phenyl group to yield a sulfate or sulfonate ester. When the sulfate or sulfonate ester is administered to a subject, the ester is cleaved, enzymatically or non-enzymatically, reductively or hydrolytically, to reveal the anionic group. Such an ester can be cyclic, e.g., a cyclic sulfate of sultone, or two or more anionic moieties may be esterified through a linking group.

An anionic group can be esterified with moieties (e.g., acyloxymethyl esters) which are cleaved to reveal an intermediate compound which subsequently decomposes to yield the active compound. In another embodiment, the prodrug is a reduced form of a sulfate or sulfonate, e.g., a thiol, which is oxidized win vivow to the therapeutic compound. Furthermore, an anionic moiety can be esterified to a group which is actively transported *in vivo*, or which is selectively taken up by target organs. The ester can be selected to allow specific targeting of the therapeutic moieties to the brain.

Carrier molecules may also be used to transport the active compounds to the brain. The carrier molecule may include a moiety capable of targeting the therapeutic compound to the brain by either active or passive transport. Illustratively the carrier molecule may include a redox moiety. Many targeting moieties are known, including those disclosed in patents to Bodor 4,540,564, 5,389,623, and 5,525,727, as well as asialoglycoproteins, such as those disclosed in Wu et al U.S. Patent 5,166,320. Other carrying moieties include ligands which are transported into cells via receptor-mediated endocytosis.

Other means for carrying a prodrug or an active agent across the blood-brain barrier involve coupling either a prodrug or an active agent to a carrier. These carriers include fatty acids, inositol, 1,4-dihydropyridine, lipids, proteins, and peptides. The proteins and peptides can be synthetic or naturally occurring. The term peptide is intended to include small proteins and particularly those molecules having on the order of about 100 amino acids or fewer. Examples of proteins useful as carriers include antibodies specific for receptors within the brain, albumin, insulin, drugs, or growth factors.

Fatty acid carriers preferably have between about 16 and 26 carbon atoms, and preferably between about 20 and 24 carbon atoms. The length, degree of saturation, and whether the fatty acid is naturally occurring in the brain affects the ability of the fatty acid to serve as a carrier to deliver the agent across the blood brain barrier to an active site in the brain. Fatty acids which are partially unsaturated and occur naturally in the brain are particularly preferred as carriers. Fatty acids which occur naturally in the brain include those with 16 carbon atoms and 0, 1 or 2 double bonds. It has been found that C18:3 is superior in its ability to deliver a compound across the blood brain barrier, and 4, 7, 10, 13, 16, 19 docosahexaenoic acid has also been found to be particularly useful in delivering agents across the blood brain barrier.

Branched chain fatty acids having between 16 and 26 carbon atoms can also be used in the present invention. A hydrogen atom of the foregoing fatty acids can be replaced with a methyl, ethyl or isopropyl substituent at various positions along the carbon chains.

Other carrier include the naturally occurring polyisoprenoids (dolicols) and analogs thereof.

The active agent can be coupled to the fatty acid via a group capable of being attached directly or indirectly to the hydroxyl group of the fatty acid. The hydroxyl group of the fatty acid can form, for example, an ester of amide bond with the agent. A hydroxyl group or amino group of the active agent can form a bond with the fatty acid. A variety of reactions can be used involving reacting the agent or a protected derivative thereof with the corresponding fatty acid carrier or an activated derivative thereof. A free hydroxyl group can form an ester bond with the fatty acid or activated derivative thereof, and the free amino group can form an amide bond with the fatty acid or activated derivative thereof.

The targeting and prodrug strategies described above can be combined to produce a compound that can be transported as a prodrug to a desired site of action and then unmasked to reveal an active compound.

In another embodiment of the present invention, the active agent is contained within a compatible biodegradable polymer in the form of microspheres. As used herein the term "microspheres" includes microcapsules, nanocapsules, and nanospheres.

Microcapsules and microspheres are conventionally free flowing powders consisting of spherical particles of 2 millimeters or less in diameter, usually 500 microns or less in diameter. Particles less than 1 micron are conventionally referred to as nanocapsules of nanospheres. For the most part, the difference between a microcapsule and a nanocapsule, or a microsphere and a nanosphere, is size. Generally, there is little, if any, difference between the internal structure of the two.

The microcapsule or nanocapsule has its encapsulated agent centrally located within a membrane. This membrane may be termed a wall-forming polymeric material. Because of their internal structure, permeable microcapsules designed for controlled-release applications release their agent at a constant rate (called a "zero order" rate of release).

In addition, the microspheres may encompass "monolithic" and similar particles in which the active agent is dispersed throughout the particle. That is, the internal structure is a matrix of the bioactive agent and a polymer excipient. Usually such particles release their bioactive agents at a declining rate (a "first order" rate of release). However, these particles may be designed to release active agents within the matrix at a near zero order rate. Thus, as used in the present invention, microspheres also include microparticles in general which have an internal structure comprising a matrix of bioactive agent and polymer excipient.

One particularly useful polymer for preparing a microcapsule or microsphere according to the present invention is poly(lactide-co-glycolide). This polymer is similar to materials used in the manufacture of conventional resorbable sutures. This polymer is biocompatible with the tissues of the central nervous system. Additionally, this polymer is biodegradable within the tissues of the central nervous system without producing any toxic by-products of degradation. A still further advantage of this material is the ability to modify the duration of drug release by manipulating the polymer's biodegradation kinetics, i.e., by modifying the ratio of lactide and glycolide in the polymer to deliver the glucose-affecting molecules to specific regions of the brain at a controlled rate over a predetermined period of time. Microspheres made with this polymer, thus, serve two functions: they protect the active agents from degradation, and they release the active agents at a controlled rate over a predetermined time. Although this specific polymer meets the criteria necessary for implantation within the central nervous system, other biocompatible, biodegradable polymers and copolymers having properties which are similar to those named properties of poly(lactide-co-glycolide) may be substituted therefor.

Pharmaceutical compositions for administration according to the present invention can comprise at least one agent according to the present invention in a pharmaceutically acceptable form, optionally combined with a pharmaceutically acceptable carrier. These compositions can be administered by any means that achieve their intended purposes. Amounts and regimens for the administration of a composition according to the present invention can be determined readily by those with ordinary skill in the art of treating memory loss or senile dementia.

For example, administration can be by parenteral, such as subcutaneous, intravenous, intramuscular, intraperitoneal, transdermal, or buccal routes. Alternatively or concurrently, administration can be by the oral route, transdermally, transmucosally, or rectally. While oral dosage is preferred, administration by suppositories may be useful. The dosage administered depends upon the age, health and weight of the recipient, type of previous or concurrent treatment, if any, frequency of the treatment, and the nature of the effect desired.

Preferably, the compounds in the form of prodrugs can be administered parenterally (intravenously, intraperitoneally, or intramuscularly) by simply combining a therapeutic amount of the prodrug with any pharmaceutically acceptable inert parenteral carrier. With respect to oral administration, since the compounds of the present invention are absorbed effectively through the intestine, but are highly sensitive compounds, they are preferably administered in an enteric coated medium. Of course, the carrier material in either case must be inert, i.e., non-oxidizing, in nature. The reason for this is that if the compounds of the present invention are contacted with an oxidizing agent prior to entry into the bloodstream, the compound will immediately be oxidized to the active form, and will not cross the blood brain barrier. The only limitation on the enteric coating is that it preserve the prodrug from dissolution until it reaches the small intestine, and that the enteric coating medium be inert, i.e., non-oxidizing. Consequently, most conventional enteric coating compositions can be used.

Compositions within the scope of this invention include all compositions comprising at least one agent according to the present invention in an amount that is safe and effective to achieve its intended purpose. While individual needs vary, determination of optimal ranges of effective amounts of each component is within the skill of the art. Typical dosages can comprise from about 0.0001 to about 100 mg/kg body weight daily.

The effective amounts of agents for improving brain glucose utilization can be readily determined. For *in vivo* prevention or reduction of memory loss and dementia, the presently preferred daily dosage is between about 1 microgram and about 100 grams of the active agent. Of course, the actual preferred amount of agent to be administered varies according to the particular form of the agent, whether it is the agent *per se* or an analog thereof, the particular composition formulated, and the mode of administration.

Administration can be conducted continuously or periodically within the maximum dose tolerated by the individual patient. Of course, optimal administration rates for a given set of conditions can be ascertained by those skilled in the art using conventional dosage administration tests.

Brain glucose utilization is improved where the administration of the agent activates the insulin transduction process so that the net effect is to increase the sensitivity or responsiveness of the insulin signal. Surprisingly, it is a discovery of the present invention that improving brain glucose utilization in non-diabetic patients does not affect the glycemic profile of these patients. That is, the agent only improves glucose utilization in the brain and does not affect the level of peripheral glycemia, because delivery of the agent solely to the central nervous system does not affect the peripheral blood glucose.

The preferred dose of an effective compound is from about 0.01 mg to about 4 grams, administered up to eight times daily depending upon the degree of impairment of glucose utilization in the individual patient. Typically, the dose is from about 0.1 mg to about 1 gram administered up to four times daily, and the preferred dose is in the rage of about 0.1 mg to about 400 mg, administered once or twice daily.

The insulin sensitizer of the present invention may be co-administered with other agents designed to improve mental performance, particularly agents that increase energy utilization. These agents include acetyl-carnitine, other forms of carnitine, and cerebral enhancers such as cognex.

The compounds of the present invention can be administered in the form of pharmaceutically acceptable compositions, that is, with the active ingredient mixed with or encapsulated in a pharmaceutically acceptable carrier. Compositions within the scope of the invention, thus, include compositions wherein the active component is present in an effective amount to achieve its intended purpose. Determination of the effective amounts is well within the skill in the art.

In addition to the compounds of the present invention, these pharmaceutical compositions may contain suitable pharmaceutically acceptable carriers comprising excipients and auxiliaries which facilitate processing of the active compounds into preparations which can be used pharmaceutically. Preferably, the preparations, particularly those which can be administered orally and which can be used for the preferred type of administration, such as tablets, dragees and capsules, and also preparations which can be administered rectally, such as suppositories, as well as suitable solutions for administration by injection or orally, contain from about 0.1 to 99%, and preferably from about 1-85% of the active ingredient, together with a suitable excipient.

The pharmaceutical preparations of the present invention are manufactured in a manner which is itself known, for example, by means of conventional mixing, granulating, dragee-making, dissolving, or lyophilizing processes. Thus, pharmaceutical preparations for oral use can be obtained by combining the active compounds with solid excipients, optionally grinding a resulting mixture, and processing the mixture of granules, after adding suitable auxiliaries, if desired or necessary, to obtain tablets of dragee cores.

Examples of suitable excipients include lactose, sucrose, mannitol, sorbitol, cellulose preparations, calcium phosphates, binders, such as starch paste from maize starch, wheat starch, rice starch, potato starch, gelatin, tragacanth, methyl cellulose, hydropropylmethlycellulose, sodium carboxymethylcellulose and/or polyvinyl pyrrolidone. If desired, disintegrating agents may be added, such as the above-mentioned starches, as well as carboxymethyl starch, cross-linked polyvinyl pyrrolidone, agar, alginic acid, sodium alginate, and the like.

Auxiliaries include flow-regulating agents and lubricants, such as silica, talc, stearic acid or salts thereof and/or polyethylene glycol. Dragee cores are provided with suitable coatings which, if desired, are resistant to gastric juices. For this purpose, concentrated sugar solutions may be used, which may optionally contain gum arabic, talc, polyvinyl pyrrolidone, polyethylene glycol and/or titanium dioxide, lacquer solutions and suitable organic solvents or solvent mixtures.

In order to produce coatings resistant to gastric juices, solutions of suitable cellulose preparations, such as acetyl cellulose phthalate or hydroxypropylmethyl cellulose phthalate are used. Dyestuffs or pigments may be added to the tablets or dragee coatings, for example, for identification or in order to characterize different combinations of active compound doses.

Other pharmaceutical preparations which can be used orally include push-fit capsules made of gelatin, as well as soft, sealed capsules made of gelatin and a plasticizer, such as glycerol or sorbitol. The push-fit capsules can contain the active compounds in the form of granules which may be mixed with filler, such as lactose, binder, such as starches, and/or lubricants, such as talc or magnesium stearate and, optionally, stabilizers. In soft capsules, the active compounds are preferably dissolved or suspended in suitable liquids, such as fatty oils, liquid paraffin, or liquid polyethylene glycols. In addition, stabilizers may be added.

Possible pharmaceutical preparations which can be used rectally include, for example, suppositories, which consist of a combination of the active compounds with a suppository base. Suitable suppository bases are, for example, natural or synthetic triglycerides, paraffin hydrocarbons, polyethylene glycols, or higher alkanols. In addition, it is also possible to use gelatin rectal capsules which consist of a combination of the active compounds with a base. Possible base materials include, for example, liquid triglycerides, polyethylene glycols, or paraffin hydrocarbons.

Suitable formulations for parenteral administration include aqueous suspensions of the active ingredients, as well as appropriate oily injection suspensions. Suitable lipophilic vehicles include fatty oils such as sesame oil, or synthetic fatty acid esters, such as ethyl oleate or triglycerides. Aqueous injection suspensions may contain substances which increase the viscosity of the suspension, such as sodium carboxymethyl cellulose, sorbitol and/or dextran. Optionally, the suspension may also contain stabilizers.

The compounds of the present invention may be administered in a variety of convenient forms, orally, parenterally, rectally, or percutaneously to treat dementia. The dosage required for each patient may vary widely, depending upon the degree of dementia and the individual patient response. However, in general, a dosage of from about 0.001 to about 100 mg/kg of body weight is appropriate for most patients.

The foregoing description of the specific embodiments will so fully reveal the general nature of the invention that others can, by applying current knowledge, readily modify and/or adapt for various applications such specific embodiments without undue experimentation and without departing from the generic concept, and, therefore, such adaptations and modifications should and are intended to be comprehended within the meaning and range of equivalents of the disclosed embodiments. It is to be understood that the phraseology or terminology employed herein is for the purpose of description and not of limitation. The means, materials, and steps for carrying out various disclosed functions may take a variety of alternative forms without departing from the invention. Thus the expressions "means to..." and "means for...", or any method step language, as may be found in the specification above and/or in the claims below, followed by a functional statement, are intended to define and cover whatever structural, physical, chemical or electrical element or structure, or whatever method step, which may now or in the future exist which carries out the recited function, whether or not precisely equivalent to the embodiment or embodiments disclosed in the specification above, i.e., other means or steps for carrying out the same function can be used; and it is intended that such expressions be given their broadest interpretation.

References to known method steps, conventional method steps, known methods or conventional methods is not in any way an admission that any aspect, description, or embodiment of the present invention is disclosed, taught, or suggested in the relevant art.

### REFERENCES

Beagley et al, J. Neurochem. 55:1222 (1990)
Blum-Degen et al, J. Neural. Transm. Suppl. 46:139-147 (1995)
Bodor et al, in Peptide-Based Drug Design: Controlling Transport and Metabolism, Taylor et al (Eds) Chapter 14 (1995)
Bodor et al, Science 214:1370-1372 (1981)
Bodor et al, Pharmacology and Therapeutics 19(3) :337-386 (1983)
Bodor et al, Science 257:1698-1700 (1992)
Buckle et al, Bioorganic and Medicinal Chemistry Letters 6:2121- 2126 (1996a)
Buckle et al, Bioorganic and Medicinal Chemistry Letters 6:2121-2126 (1996b)
Cantello et al, J. Med. Chem. 22 1977 3885 (1994)
Clarke et al, J. Biol. Chem. 259: 11672-11675 (1984)
Cullingford, J. Neurochem., 70:1366-1375 (1998)
Dienel et al, J. Neurochem. 59:1430-1436 (1992)
Dore et al, Neuroscience, 80(4):1033-1040 (1997)
Doyle et al, Brain Research 684:47-55 (1995)
Duelli et al, Int. J. Dev. Neurosci. 12(8):737-743 (1994)
Hasselbalch et al., in Diabetes 48:(10): 1915-1921, October, 1999,
Granneman et al J. Neuroscience Res. 51:563-573 (1998)
Hoyer et al, Ann. NY Acad. Sci. 777:374-379 (1996)
Kitamura et al, Biochem. Phys. Res. Commun. January 27, 254 (3) :582-6
Kitamura et al., Neurosci. Lett. 262(2):129-32, March 5, 1999
Kumagai, in Diabetes Metab. Res. Rev. 15(4):261-273, Jul-Aug, 1999
Mukherjie et al, Nature 386:407-410 (1997)
Nakagama et al, Neurobiology of Ageing 17(6) :849-857 (1996)
Neuwelt et al, Fed. Proc. 43:214 (1984)
Pardridge, Endocrinol. Rev. 7:314 (1986)
Petrova et al., Proc. Natl. Acad. Sci. USA 96(8):4668-73, April 13, 1999
Poplack et al, Neurobiology of Cerebrospinal Fluid, J.H. Wood (Ed), Plenum Press (New York, 1981), pp. 561-578
Prokai et al, J. Am. Chem. Soc. 116:2643-2644 (1994)
Smith and Sokoloft in The Aging Brain
Tsuzuki et al, Biochem. Pharmacol. 41:R5 (1991)

## Claims

1. Use of an agent that improves insulin sensitivity of peripheral tissues in non-insulin dependent diabetic patients and is useful in treating non-insulin dependent diabetes
for the preparation of a pharmaceutical composition
for treating Alzheimer's disease, memory loss, senile dementia, or dementia, when the symptoms of reduced mental performance are not related to non-insulin dependent diabetes or a state of general impaired glucose tolerance,
with the proviso that the use is not the use of a thiazolidinedione for the preparation of a pharmaceutical composition for treating Alzheimer's disease.

2. Use according to claim 1, wherein the agent is selected from the group consisting of insulin sensitizers.

3. Use according to claim 2, wherein the agent is a thiazolidinedione.

4. Use according to claim 3, wherein the thiazolidinedione is selected from the group consisting of rosiglitazone, pioglitazone, darglitazone, troglitazone and englitazone.

5. Use according to claim 2, wherein the agent is an oxyzolidinedione.

6. Use according to claim 1, wherein the agent activates the PPAR gamma receptor, has agonist or partial agonist activity at the PPAR gamma receptor.

7. Use according to claim 6, wherein the agent is a substituted 4-hydroxy-phenylalcanoic acid derivative.

8. Use according to claim 6, wherein the agent is a non-thiazolidinedione, non-oxyzolidinedione.

9. Use according to claim 1, wherein the agent selectively activates one of the sub-types of the human PPAR gamma receptor, a RxR receptor that forms a heterodimer with a PPAR gamma receptor, wherein the agent is a combination of a PPAR gamma activator and an RxR receptor activator, or interacts with a PPAR alpha receptor or a PPAR delta receptor or with the insulin transduction process so that the net effect is to increase the sensitivity or responsiveness of the insulin signal.

10. Use according to claim 1, wherein the composition further comprises at least one agent, which improves mental performance, selected from the group consisting of carnitine, acetyl-carnitine and cerebral enhancers.

11. Use according to anyone of claims 1-10, wherein the agent is in the form of a prodrug.

12. Use according to claim 11, wherein the agent is provided in the form of an acid addition salt.

13. Use according to claim 11, wherein the agent is linked through a spacer to a dihydropyridine redox moiety.

14. Use according to anyone of claims 1-13, wherein the composition is in a form that enables the agent to cross the blood brain barrier.

15. Use according to claim 14, wherein the composition comprises an effective amount of egressin to enable delivery of the agent across the blood brain barrier.

16. Use according to claim 14, wherein the composition is formulated as a non-ionic compound.

17. Use according to claim 14, wherein the composition is microencapsulated in a poly (lactide-co-glycolide) biodegradable polymer.

## Patentansprüche

1. Verwendung eines Wirkstoffs, der die Insulinsensitivität von peripheren Geweben bei Patienten mit nicht-insulinabhängigem Diabetes verbessert und bei der Behandlung von nicht-insulinabhängigem Diabetes von Nutzen ist,
zur Herstellung einer pharmazeutischen Zusammensetzung
zum Behandeln der Alzheimer-Krankheit, von Gedächtnisverlust, seniler Demenz oder Demenz, wenn die Symptome des verminderten mentalen Leistungsvermögens nicht mit nicht-insulinabhängigem Diabetes oder einem Zustand einer allgemein gestörten Glucosetoleranz in Beziehung stehen,
mit der Maßgabe, dass es sich bei der Verwendung nicht um die Verwendung eines Thiazolidindions zur Herstellung einer pharmazeutischen Zusammensetzung zum Behandeln der Alzheimer-Krankheit handelt.

2. Verwendung gemäß Anspruch 1, wobei der Wirkstoff aus der aus Insulinsensitizern bestehenden Gruppe ausgewählt ist.

3. Verwendung gemäß Anspruch 2, wobei es sich bei dem Wirkstoff um ein Thiazolidindion handelt.

4. Verwendung gemäß Anspruch 3, wobei das Thiazolidindion aus der aus Rosiglitazon, Pioglitazon, Darglitazon, Troglitazon und Englitazon bestehenden Gruppe ausgewählt ist.

5. Verwendung gemäß Anspruch 2, wobei es sich bei dem Wirkstoff um ein Oxyzolidindion handelt.

6. Verwendung gemäß Anspruch 1, wobei der Wirkstoff den PPAR-gamma-Rezeptor aktiviert, agonistische oder teilweise agonistische Aktivität am PPAR-gamma-Rezeptor aufweist.

7. Verwendung gemäß Anspruch 6, wobei es sich bei dem Wirkstoff um ein substituiertes 4-Hydroxy-phenylalkansäurederivat handelt.

8. Verwendung gemäß Anspruch 6, wobei es sich bei dem Wirkstoff um ein Nicht-Thiazolidindion, Nicht-Oxyzolidindion handelt.

9. Verwendung gemäß Anspruch 1, wobei der Wirkstoff selektiv einen der Subtypen des humanen PPAR-gamma-Rezeptors und/oder einen RxR-Rezeptor, der ein Heterodimer mit einem PPAR-gamma-Rezeptor bildet, aktiviert, wobei der Wirkstoff eine Kombination eines PPAR-gamma-Aktivators und eines RXR-Rezeptoraktivators ist oder mit einem PPAR-alpha-Rezeptor oder einem PPARdelta-Rezeptor oder mit dem Insulin-Transduktionsprozess so interagiert, dass die Nettowirkung darin besteht, dass die Sensitivität oder die Empfindlichkeit des Insulinsignals gesteigert wird.

10. Verwendung gemäß Anspruch 1, wobei die Zusammensetzung weiter mindestens einen Wirkstoff, der das mentale Leistungsvermögen verbessert und aus der aus Carnitin, Acetylcarnitin und Mitteln zur Verbesserung der zerebralen Funktion bestehenden Gruppe ausgewählt ist, umfasst.

11. Verwendung gemäß einem der Ansprüche 1-10, wobei der Wirkstoff in Form eines Pro-Pharmakons vorliegt.

12. Verwendung gemäß Anspruch 11, wobei der Wirkstoff in Form eines Säureadditionssalzes bereitgestellt wird.

13. Verwendung gemäß Anspruch 11, wobei der Wirkstoff durch einen Spacer an eine Dihydropyridin-Redox-Komponente gekoppelt ist.

14. Verwendung gemäß einem der Ansprüche 1-13, wobei die Zusammensetzung in einer Form vorliegt, welche den Wirkstoff in die Lage versetzt, die Blut-Hirn-Schranke zu durchqueren.

15. Verwendung gemäß Anspruch 14, wobei die Zusammensetzung eine wirksame Menge von Egressin umfasst, um die Abgabe des Wirkstoffs über die Blut-Hirn-Schranke hinweg zu ermöglichen.

16. Verwendung gemäß Anspruch 14, wobei die Zusammensetzung als nicht-ionische Verbindung formuliert ist.

17. Verwendung gemäß Anspruch 14, wobei die Zusammensetzung in einem biologisch abbaubaren Poly(Lactid-co-Glycolid)-Polymer mikroverkapselt ist.

## Revendications

1. Utilisation d'un agent qui améliore la sensibilité à l'insuline des tissus périphériques chez les patients atteints de diabète non insulino-dépendant et est utile dans le traitement de diabètes non insulino-dépendants
pour la préparation d'une composition pharmaceutique
pour traiter la maladie d'Alzheimer, la perte de mémoire, la démence sénile, ou la démence, lorsque les symptômes de performance mentales réduites ne sont pas liées à des diabètes non insulino-dépendants ou à un état d'intolérance au glucose générale,
à la condition que l'utilisation ne soit pas l'utilisation d'une thiazolidinedione pour la préparation d'une composition pharmaceutique pour traiter la maladie d'Alzheimer.

2. Utilisation selon la revendication 1, dans laquelle l'agent est choisi parmi les insulinosensibilisateurs.

3. Utilisation selon la revendication 2, dans laquelle l'agent est une thiazolidinedione.

4. Utilisation selon la revendication 3, dans laquelle la thiazolidinedione est choisie parmi la rosiglitazone, la pioglitazone, la darglitazone, la troglitazone et l'englitazone.

5. Utilisation selon la revendication 2, dans laquelle l'agent est une oxyzolidinedione.

6. Utilisation selon la revendication 1, dans laquelle l'agent active le récepteur PPAR gamma, a une activité agoniste ou agoniste partiel au niveau du récepteur PPAR gamma.

7. Utilisation selon la revendication 6, dans laquelle l'agent est un dérivé d'acide 4-hydroxyphénylalcanoïque substitué.

8. Utilisation selon la revendication 6, dans laquelle l'agent est un composé non-thiazolidinedione, non oxyzolidinedione.

9. Utilisation selon la revendication 1, dans laquelle l'agent active sélectivement un des sous-types du récepteur PPAR gamma humain, un récepteur RxR qui forme un hétérodimère avec un récepteur PPAR gamma, où l'agent est une combinaison d'un activateur de PPAR gamma et d'un activateur de récepteur RxR, ou interagit avec un récepteur PPAR alpha ou un récepteur PPAR delta ou avec le procédé de transduction de l'insuline de telle sorte que l'effet net est l'augmentation de la sensibilité ou de la réactivité du signal de l'insuline.

10. Utilisation selon la revendication 1, dans laquelle la composition comprend en outre au moins un agent, qui améliore les performances mentales, choisi parmi la camitine, l'acétylcarnitine et les stimulants cérébraux.

11. Utilisation selon l'une quelconque des revendications 1 à 10, dans laquelle l'agent est sous la forme d'une prodrogue.

12. Utilisation selon la revendication 11, dans laquelle l'agent est fourni sous la forme d'un sel d'addition acide.

13. Utilisation selon la revendication 11, dans laquelle l'agent est lié via un espaceur à une entité redox dihydropyridine.

14. Utilisation selon l'une quelconque des revendications 1 à 13, dans laquelle la composition est sous une forme qui permet à l'agent de franchir la barrière hémato-encéphalique.

15. Utilisation selon la revendication 14, dans laquelle la composition comprend une quantité suffisante d'egressine pour permettre la délivrance de l'agent au-travers de la barrière hémato-encéphalique.

16. Utilisation selon la revendication 14, dans laquelle la composition est formulée sous la forme d'un composé non ionique.

17. Utilisation selon la revendication 14, dans laquelle la composition est micro-encapsulée dans un polymère poly(lactide-co-glycolide) biodégradable.
